# EUROPEAN PATENT SPECIFICATION

(11) **EP 2 470 504 B1**
(45) Date of publication and mention of the grant of the patent: **16.10.2013**
(21) Application number: 10847777.9
(22) Date of filing: 30.04.2010
(51) Int. Cl.: C07D 211/46

(54) **SYNTHESIS OF PROPIVERINE HYDROCHLORIDE**
SYNTHESE VON PROPIVERINHYDROCHLORID
SYNTHÈSE DE CHLORHYDRATE DE PROPIVÉRINE

(30) Priority: 15.03.2010 IN CH06742010
(43) Date of publication of application: 04.07.2012
(73) Proprietor: Andagar Ramakrishna, Ramesha, Bangalore 560 106 (IN); Roy, Anjan Kumar, Bangalore 560 106 (IN)
(72) Inventor: Andagar Ramakrishna, Ramesha, Bangalore 560 106 (IN); Roy, Anjan Kumar, Bangalore 560 106 (IN)
(74) Representative: Ellis, Michael James
(86) International application number: PCT/IB2010/051911
(87) International publication number: WO 2011/114195

(56) References cited:
- KR-A- 20050 011 139
- KR-A- 20050 011 139
- BÜCHI, J. ET AL.: "40. Synthese und spasmolytische Wirkung einiger Esterather von disubstituierten Glykolsauren", HELVETICA CHIMICA ACTA, vol. 34, no. 40, 1951, pages 373-381, XP002677809,
- BUCHI J. ET AL.: 'Synthesis and spasmolytic action of ester-ethers of di-substituted glycolic acids' HELVETICA CHIMICA ACTA vol. 34, 1951, pages 373 - 381

## Description

### Technical Field

The present invention provides a process for synthesis of Propiverine Hydrochloride. Particularly the present invention provides a process for the synthesis of Propiverine hydrochloride by etherification-esterification followed by trans esterification.

More particularly the present invention provides a process for synthesis of Propiverine Hydrochloride by esterification and etherification is done in single step.

### Background of invention

Propiverine Hydrochloride is a new class of drug used in treating "Urinary Incontinence". Propiverine is one of the most frequently prescribed drugs in treatment of urinary incontinence. Comparable drugs which are available in the market are oxybutynin, tolterodine-tartrate and trospium chloride. Propiverine Hydrochloride has dual mode of action. First being, inhibition of calcium influx and modulation of intracellular calcium in urinary bladder smooth muscle cells causing musculotropic spasmolysis. Later being Inhibition of the efferent neurotransmission of the nervus pelvicus due to anticholinergic action. Propiverine Hydrochloride has been shown to be efficacious in symptomatic treatment of urinary incontinence and increased urinary frequency and urgency as may occur in patients with overactive bladder syndrome or neurogenic detrusor overactivity (detrusor hyperreflexia) from spinal cord injuries, e.g. transverse lesion paraplegia. Propiverine Hydrochloride is well tolerated by patients, specially on a long-term basis. All adverse events are dose-dependent, transient and reversible.

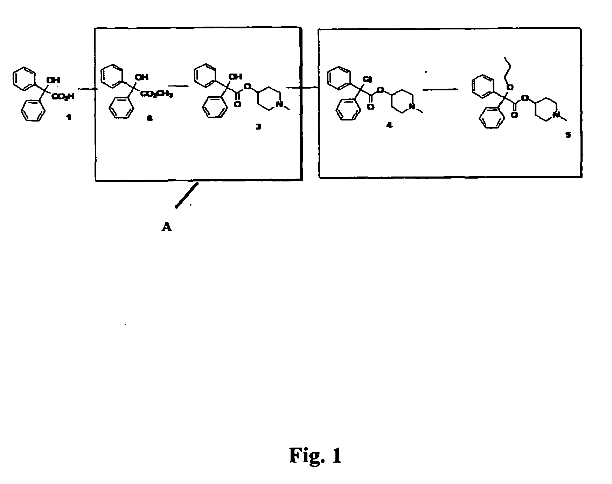

Synthesis of Propiverine Hydrochloride. has been described in several patents. Initial patent was published by Starke, Thomas and Friese (Dutch Patent No: 106 643,) in 1974. In the process described in this patent, benzillic acid is converted to methyl ester and then it is converted to hydroxyl N-methylpiperidino ester intermediate 3 (Figure 1). This reaction is always low yielding due to competing hydrolysis mediated by adjacent hydroxyl group of methyl benzillate. More over the conversion of intermediate 4 to Propiverine base 5 is also low yielding due to competing hydrolysis of the tert chloro present in 4.

A the second patent to Luo, M, Ma X, Luo P (No: CN 1285348,) the synthesis in almost similar reaction sequence as in the other mentioned patent.

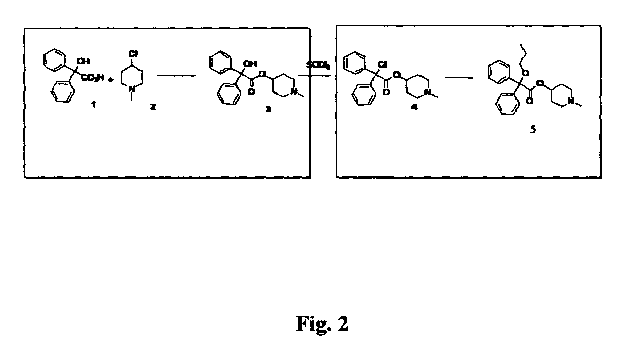

In this patent benzillic acid is converted to Hydroxy ester intermediate 3 (Figure 2) directly. During which one of the main problem is the elimination of chloro of the piperidine moiety, thereby lower yield in the process. In the second step, there is a competing hydrolysis of the intermediate 4 with moisture to intermediate 3, thereby lowering the yield.

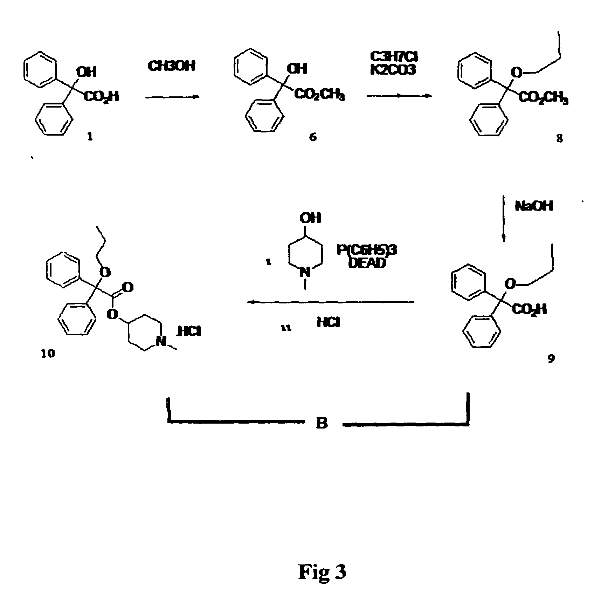

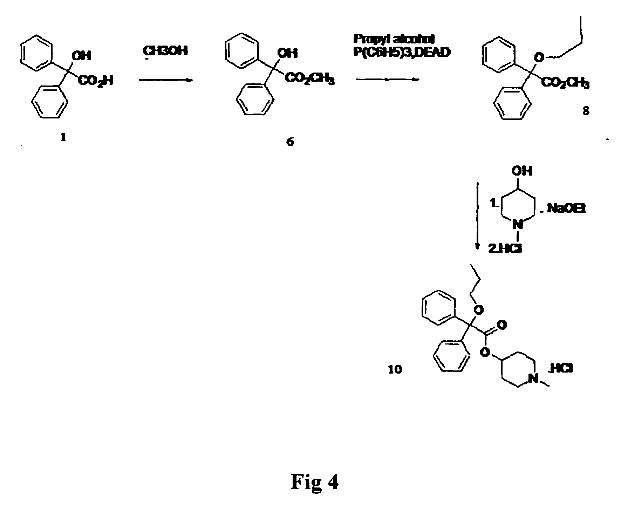

As per Korean patent KR20050011138A, there is slight change in the process described. Methyl benzillate 6 is converted to propylmethyl benzillate 7 as in Figure 3. Which is then hydrolyzed to benzillic acid propyl ether 8. Which is then finally converted to Propiverine base using Mitsunobu reaction. Mitsunobo reaction generates lots of waste in the form of triphenyl oxide and the handling of diethylazidodicarboxylate (DEAD) is dangerous. A slightly modified scheme of the above patent is presented in Korean patent KR20050011139A (Figure 4). The propylmethyl benzillate (working example 1) as well as the O-n-propylbenzilic acid n-propyl ester (working example 5) are prepared using Mitsunobu reaction. In working example 5, Propiverine hydrochloride is synthesized from the O-n-propylbenzilic acid n-propyl ester intermediate by reaction with 4-hydroxy-1-methyl piperidine in the presence of sodium ethoxide. Again the use of Mitsunobu reaction (Figure 4), is not cost effective and involves waste generation

In the present invention a new and simplified synthetic route for the synthesis of this Propiverine Hydrochloride is hereby disclosed which overcomes the drawbacks of the prior art patents.

It is an object of the present invention to provide new process for the synthesis of Propiverine Hydrochloride.

Another object of the present invention is to provide a process for synthesis of Propiverine hydrochloride by trans esterification catalyzed by potassium t-butoxide.

Still another object of the present invention is to provide a process for synthesis by etherification followed by esterification to get the critical intermediate.

Yet another object of the present invention is to provide a process to give High over all yield.

Still further object of the present invention is to provide a pure product by the process of invention.

### Brief Description of drawings

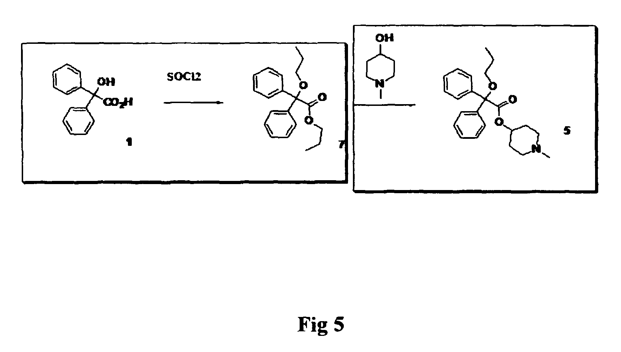

In the drawing accompanying this specification Figure 1 represents a prior art with two step esterification as per Dutch patent (DP 106643). In the figure (1) is Benzillicacid, (6) is Benzillic acid ester and (3) is the hydroxyl ester intermediate. Combination step (A) refers to a low yielding stage due to presence of the 2-hydroxyl group. This step (A) then leads to formation of propiverine chloro base (5) via a chloro intermediate (4). Figure 2 represents another prior art esterification as per Chinese patent CN 1285348. In this figure Benzillicacid (1) reacts with 4-chloro-N methyl piperidine (2) to give hydroxyl ester intermediate (3). This stage further under catalytic action of SOCl₂ leads to Chloro ester intermediate (2) which under action of propyl alcohol leads to formation of propiverine base (5). Figure 3 and 4 represent prior art as per Korean patent KR20050011138 and KR20050011139 respectively. In figure 3, (1) is benzillic acid leading to formation of methyl benzillate (6) under methyl alcohol and acid. (8) is propyl methylbenzillate which under alkaline hydrolysis forms benzillic acid propyl ether (9). This leads to stage (B) which is representative of a Mitsunobu reaction which generates Ph₃PO which has a very low atom economy. In figure 4, (1) is Benzillic acid which under alcohol acid leads to methyl benzillate (6) and under action of propyl alcohol and diethyl azido dicarboxylate (DEAD) leads to (8) the propyl methylbenzillate and further leads to formation of propiverine HCl (10). Figure 5 represents the synthesis of Propiverine Hydrochloride (10) as an embodiment of the present invention. In this figure (1) is benzillicacid. This under action of n-propyl alcohol, SOCl₂ and organic acid leads to formation of 2,2-diphenyl-2-propyloxy propyl acetate (7) when. This compound (7) under action of a catalyst leads to propiverine base (5) which when chlorinated yields propiverine HCl.

### Summary of Invention

Accordingly the present invention provides a process as defined in claim 1 for synthesis of propiverine hydrochloride.

In the present invention the catalyst used may be potassium t butoxide or sodium t-butoxide along with phase transfer catalysts.

In an embodiment of the present invention the phase transfer catalyst may be selected from a group consisting of Tetra butyl ammonium bromide , tetraphosphonium bromide ,polyethylene glycol, ammonium or phosphonium salts).

In another embodiment of the present invention the inert gas used may be nitrogen, argon, helium.

In still another embodiment of the present invention the stirring may be done for a duration of 1 to 3 days.

In yet another embodiment of the present invention the inorganic chloride may be selected from a group of ammonium chloride to neutralize the reaction medium.

In still another embodiment of the present invention the first organic solvent may be toluene,

In still another embodiment of the present invention the second organic solvent may be acetone.

Preferably, the present invention provides a process for synthesis of 2,2-diphenyl-2-propyloxy propyl acetate 7 which comprises using benzillic acid 1 and n-propyl alcohol in the presence of one or more of an inorganic acid reagent, an organic reagent, an inorganic salt, an organic salt, the said process further comprising refluxing benzillic acid 1 with n-propyl alcohol in the presence of said one or more of an inorganic acid reagent, an organic reagent, an inorganic salt, an organic salt, in the ration in a range of 10-200% mole ratio, to produce 2,2-diphenyl-2-propyloxy propyl acetate 7 as viscous liquid.

In another embodiment of the present invention inorganic acid may be sulphuric acid

In yet another embodiment the organic reagent may be methane sulphuric acid

In still another embodiment the inorganic salt may be phosphorous oxychloride.

In still further embodiment of the invention the organic salts may be thionyl chloride.

In an embodiment of the present invention the yield of the ester is at least 90%.

In an embodiment of the present invention Propiverine hydrochloride may be used to treat Urinary Incontinence

### Detailed Description of Invention

In the synthetic scheme disclosed herein (Figure 5), benzillic acid 1 is directly converted to 2,2-diphenyl-2-propyloxy propyl acetate 7 from the known standard method. This does not involve any complicated reactions like the prior art method of Mitsunobu Reaction as in KR20050011138A / does not generate much waste. The intermediate thus obtained is reacted with N-Methyl 4-hydroxy piperidine in the presence of sodium t-butoxide at room temperature to get Propiverine base 5. This is then finally converted to Propiverine hydrochloride.

Synthesis of N-di-propyl ester ether (2,2,diphenyl-2-propyloxy-propyl acetate) 7.

With reference to Fig 5 2,2-diphenyl-2-propyloxy propyl acetate 7 is synthesized from classical synthetic methods using benzillic acid 1 and excess n-propyl alcohol in the presence of reagents like sulfuric acid, methane sulfuric acid, phosphorous oxychloride, PC15, thionyl chloride in almost 80-95% yield. Thus Benzillic acid 1(1 kg) is refluxed with 6 Lt of n-propyl alcohol in the presence of thionyl chloride (1.15 kg) for 10 h. After 10 h, excess n-propylalcohol is distilled under vacuum. The resultant residue was washed with 10% sodium carbonate solution to pH neutral and extracted with toluene (3 Lt). Toluene is stripped under reduced pressure to get 2,2-diphenyl-2-propyloxy propyl acetate 7 as the only product in almost 90% yield (1.23 kg) as viscous liquid.

The NMR spectra of the compound obtained is as below:
1 H NMR (CDCl₃): 7.50-7.40 & 7.35-7.25 (2 multiplets, 10H), 4.12 (t, 2H), 3.19 (t, 2H), 1.70-1.50 (3, 4H), 0.93 (t, 3H), 0.81 (t, 3H).
13C NMR (CDCl₃): 172.01, 141.30, 128.45, 127.70, 86.43, 66.95, 66.92, 23.23, 21.84, 10.66, 10.33

In another embodiment procedure, benzillic acid (1 kg) 1 is treated with PCI5 (1.82 kg) at 80-110 °C for 4 h. The residue thus obtained is refluxed with 6 L of n-propyl alcohol in the presence of thionyl chloride (1.15 kg) for 10h. After 10 h, excess n-propylalcohol is distilled under vacuum. The resultant residue was washed with 10% sodium carbonate solution to pH neutral and extracted with toluene (3 L). Toluene is stripped under reduced pressure to get 2,2-diphenyl-2-propyloxy propyl acetate 7 as the only product in almost 88 % yield (1.20 kg) as viscous liquid. The NMR spectra of the compound obtained is as above. In another experiment, Benzillic acid 1(1 kg) is refluxed phosphorous oxychloride (1.50 kg) with 6 L of n-propyl alcohol for 24 h. The reaction mixture thus obtained is worked up as before to get 2,2-diphenyl-2-propyloxy propyl acetate 7 as the only product in almost 90% yield (1.23 kg) as viscous liquid. The NMR is same as described above

Following examples are given by way of illustration only and do not limit the scope of the invention

### Synthesis of Propiverine base and Hydrochloride

### Example 1

2,2-diphenyl-2-propyloxy propyl acetate 7 (1.23 kg) is treated with N-methyl-4-piperidinol (3 kg) and catalytic amount of potassium t-butoxide (246 g) and tetrabutylammonium chloride (50 g) and stirred under nitrogen for 3 days at room temperature. At the end of 3 days TLC-Thin Layer Chromatography (1:1, acetone: chloroform) shows almost 80-90% reaction conversion. Once the TLC is acceptable, ammonium chloride (400 g) is added to bring the pH to almost neutral. Excess N-methyl-4-pipridinol is recovered under high vacuum at a pressure of about 5 mm. The residue thus obtained is extracted in toluene (4 L) and washed with water (2 L) 3 times. Toluene was removed under reduced pressure to get crude product 1.5 kg (almost 100%). Which can be converted to Propiverine hydrochloride by dissolving the residue in acetone(1:6-7) and charcolizing (10% by weight) and treating with hydrochloric acid to get almost white Propiverine HCl 0.9 kg to1.25 kg which melts at 216-218 °C, with an assay of 99.00-100.00% and HPLC purity of >99.50%.

The NMR spectra obtained for the compound is as follows
1H NMR (CDCl₃): 12.30 &12.40 (Broad singlet, 1H), 7.45-7.25 (2 multiplets, 10H), 5.14, 5.00 (2 broad multiplets, 1 H),3.23 (triplet, 2H), 2.48 (Doublet, 3H), 1.62 (quintet, 2H). 0.91 (triplet, 3H), 3.07,3.03,2.45,2.1701.88 (5 multiplets, 8H)
13C NMR (CDCl₃): 170.34, 140.80, 128.33,127.83, 128.17, 86.3,67.03,64.75,48.85, 43.45,26.57,23.18,10.64.

### Example 2

Benzillic acid (1 kg) 1 is treated with PCl5 (1.82 kg) at 80-110 °C for 4 h. The residue thus obtained is refluxed with 6 Lt of n-propyl alcohol in the presence of thionyl chloride (1.15 kg) for 10h. After 10 h, excess n-propylalcohol is distilled under vacuum. The resultant residue was washed with 10% sodium carbonate solution to pH neutral and extracted with toluene (3 L). Toluene is stripped under reduced pressure to get 2,2-diphenyl-2-propyloxy propyl acetate 7 as the only product in almost 88 % yield (1.20 kg) as viscous liquid. The NMR spectra of the compound obtained is as above. In another experiment, Benzillic acid 1(1 kg) is refluxed phosphorous oxychloride (1.50 kg) with 6 L of n-propyl alcohol for 24 h. The reaction mixture thus obtained is worked up as before to get 2,2-diphenyl-2-propyloxy propyl acetate 7 as the only product in almost 90% yield (1.23 kg) as viscous liquid. (1.23 kg) of 2,2-diphenyl-2-propyloxy propyl acetate was reacted with N-methyl-4-piperidinol (3 kg) as above and worked up as above to get about 0.88 kg of Propiverine Hydrochloride having a assay of 99-100%.

### Example 3:

2,2-diphenyl-2-propyloxy propyl acetate 7 (1.23 kg) is treated with N-methyl-4-piperidinol (3 kg) and catalytic amount of sodium t-butoxide (250 g) and tetrabutylammonium chloride (50 g) and stirred under nitrogen for 3 days at room temperature. At the end of 3 days TLC-Thin Layer Chromatography (1:1, acetone: chloroform) shows almost 80-90% reaction conversion. Once the TLC is OK, ammonium chloride (400 g) is added to bring the pH to almost neutral. Excess N-methyl-4-pipridinol is recovered under high vacuum at a pressure of about 5 mm. The residue thus obtained is extracted in toluene (4 L) and washed with water (2 L) 3 times. Toluene was removed under reduced pressure to get crude product 1.5 kg (almost 100%). Which can be converted to Propiverine hydrochloride by dissolving the residue in acetone(1:6-7) and charcolizing (10% by weight) and treating with hydrochloric acid to get almost white Propiverine HCl 0.9 kg to1.20 kg which melts at 216-218 °C, with an assay of 99.00-100.00% and HPLC purity of >99.50%.

### Example 4:

2,2-diphenyl-2-propyloxy propyl acetate **7** (1.23 kg) is treated with N-methyl-4-piperidinol (3 kg) and catalytic amount of sodium t-butoxide (250 g) and triphenylphosphonium bromide (50 g) and stirred under nitrogen for 3 days at room temperature. At the end of 3 days TLC-Thin Layer Chromatography (1:1, acetone: chloroform) shows almost 80-90% reaction conversion. Once the TLC is OK, ammonium chloride (400 g) is added to bring the pH to almost neutral. Excess N-methyl-4-pipridinol is recovered under high vacuum at a pressure of about 5 mm. The residue thus obtained is extracted in toluene (4 L) and washed with water (2 L) 3 times. Toluene was removed under reduced pressure to get crude product 1.5 kg (almost 100%). Which can be converted to Propiverine hydrochloride by dissolving the residue in acetone (1:6-7) and charcolizing (10% by weight) and treating with hydrochloric acid to get almost white Propiverine HCl 0.9 kg to1.20 kg which melts at 216-218 °C, with an assay of 99.00-100.00% and HPLC purity of >99.50%.

### Example 5

2,2-diphenyl-2-propyloxy propyl acetate **7** (1.23 kg) is treated with N-methyl-4-piperidinol (3 kg) and catalytic amount of sodium t-butoxide (250 g) and polyethylene glycol-400 (50 g) and stirred under nitrogen for 3 days at room temperature. At the end of 3 days TLC-Thin Layer Chromatography (1:1, acetone: chloroform) shows almost 80-90% reaction conversion. Once the TLC is OK, ammonium chloride (400 g) is added to bring the pH to almost neutral. Excess N-methyl-4-pipridinol is recovered under high vacuum at a pressure of about 5 mm. The residue thus obtained is extracted in toluene (4 L) and washed with water (2 L) 3 times. Toluene was removed under reduced pressure to get crude product 1.5 kg (almost 100%). Which can be converted to Propiverine hydrochloride by dissolving the residue in acetone(1:6-7) and charcolizing (10% by weight) and treating with hydrochloric acid to get almost white Propiverine HCl 0.9 kg to1.20 kg which melts at 216-218 °C, with an assay of 99.00-100.00% and HPLC purity of >99.50%.

The main advantages of the present invention are:
1. The present invention gives pure Propiverine Hydrochloride.
2. The process gives high over all yield of the end product
3. The process is easily scaled up if required

## Claims

1. A process for synthesis of Propiverine hydrochloride which comprises
reacting 2,2-diphenyl-2-propyloxy propyl acetate 7 with N-methyl-4-piperidinol and a catalyst comprising potassium or sodium t-butoxide along with a phase transfer catalyst under constant stirring in inert gas for long duration, on completion of the said reaction inorganic chloride is added to neutralize the pH, the said reaction being subjected to vacuum to remove excess N-methyl-4-piperidinol giving a residue, the said residue being further extracted in first organic solvent and washed in water, the said extraction being followed by treatment of the residue under reduced pressure to remove organic solvent to yield a raw product, being Propiverine base, the said raw product being further treated by dissolving in second organic solvent and charcolozing and treating with hydrochloric acid to yield Propiverine hydrochloride.

2. A process as claimed in Claim I wherein, the phase transfer catalyst is selected from a group consisting of Tetra butyl ammonium bromide, tetraphosphonium bromide, polyethylene glycol, ammonium or phosphonium salts.

3. A process as claimed in claim 1 or claim 2, wherein the reaction 2,2-diphenyl-2-propyloxy propyl acetate 7 with N-methyl-4-piperidinol is carried out at room temperature.

4. A process as claimed in claim 1 wherein, the inert gas is selected from nitrogen, argon, helium.

5. A process as claimed in claim 1 wherein the stirring is done for a duration of 1 to 3 days.

6. A process as claimed in claim 1 wherein, the inorganic chloride is ammonium chloride to neutralize the reaction medium.

7. A process as claimed in claim 1 wherein, the first organic solvent is toluene.

8. A process as claimed in claim 1 wherein, the second organic solvent is acetone.

9. A process as claimed in claim 1, which comprises
reacting benzillic acid 1 and n-propyl alcohol in the presence of one or more of an inorganic acid reagent, an organic reagent, an inorganic salt, or thionyl chloride, the said process further comprising refluxing benzillic acid 1 with n-propyl alcohol in the presence of said one or more of an inorganic acid reagent, an organic reagent, an inorganic salt, or thionyl chloride in a ratio in a range of 10-200% mole ratio, to produce the 2,2-diphenyl-2-propyloxy propyl acetate 7 of claim 1 as a viscous liquid, without using the Mitsunobu reaction.

10. A process as claimed in claim 9 wherein, n-propyl alcohol is mixed with dichloromethane, toluene, benzene and any combination thereof.

11. A process as claimed in claim 9 wherein inorganic acid is sulphuric acid.

12. A process as claimed in claim 9 wherein the inorganic salt is phosphorous oxychloride.

13. A process as claimed in claim 9 wherein the yield of the 2,2-diphenyl-2-propyloxy propyl acetate 7 is at least 90%.

## Patentansprüche

1. Verfahren zur Synthese von Propiverinhydrochlorid, welches umfasst, dass 2,2-Diphenyl-2-propyloxypropylacetat-7 mit N-methyl-4-piperidinol und einem Katalysator, der Kalium- oder Natrium-t-butoxid zusammen mit einem Phasentransferkatalysator enthält, unter ständigem Rühren über einen längeren Zeitraum in inertem Gas zur Reaktion gebracht wird, wobei bei der Beendigung der Reaktion ein anorganisches Chlorid zugesetzt wird, um den pH-Wert zu neutralisieren, wobei die Reaktion im Vakuum stattfindet, um überschüssiges N-methyl-4-piperidinol zu entfernen, wodurch ein Rückstand entsteht, wobei der Rückstand des Weiteren in einem ersten organischen Lösungsmittel extrahiert und in Wasser gewaschen wird, wobei nach der Extraktion die Behandlung des Rückstands unter reduziertem Druck erfolgt, um das organische Lösungsmittel zu entfernen und ein Rohprodukt zu erhalten, wobei es sich um Propiverinbase handelt, wobei das Rohprodukt des Weiteren behandelt wird, indem es in einem zweiten organischen Lösungsmittel gelöst wird, mit Aktivkohle und anschließend mit Hydrochlorsäure behandelt wird, um Propiverinhydrochlorid zu erhalten.

2. Verfahren nach Anspruch 1, wobei der Phasentransferkatalysator aus der Gruppe ausgewählt ist, die aus Tetrabutylammoniumbromid, Tetraphosphoniumbromid, Polyethylenglycol, Ammonium- oder Phosphoniumsalze besteht.

3. Verfahren nach Anspruch 1 oder Anspruch 2, wobei die Reaktion von 2,2-Diphenyl-2-propyloxypropylacetat-7 mit N-methyl-4-piperidinol bei Raumtemperatur durchgeführt wird.

4. Verfahren nach Anspruch 1, wobei das inerte Gas aus Stickstoff, Argon, Helium ausgewählt ist.

5. Verfahren nach Anspruch 1, wobei das Rühren über einen Zeitraum von 1 bis 3 Tage erfolgt.

6. Verfahren nach Anspruch 1, wobei das anorganische Chlorid zur Neutralisation des Reaktionsmediums Ammoniumchlorid ist.

7. Verfahren nach Anspruch 1, wobei das erste organische Lösungsmittel Toluol ist.

8. Verfahren nach Anspruch 1, wobei das zweite organische Lösungsmittel Aceton ist.

9. Verfahren nach Anspruch 1, welches umfasst, dass Benzilsäure-1 und n-Propylalkohol in der Gegenwart von einem oder mehreren anorganischen Säurereagenzien, organischen Reagenzien, anorganischen Salzen oder Thionylchlorid zur Reaktion gebracht wird, wobei das Verfahren des Weiteren das Kondensieren von Benzilsäure-1 mit n-Propylalkohol unter Rückfluss in Gegenwart von einem oder mehreren anorganischen Säurereagenzien, organischen Reagenzien; anorganischen Salzen oder Thionylchlorid in einem molaren Verhältnis in einem Bereich von 10-200 %, um das 2,2-Diphenyl-2-propyloxypropylacetat-7 nach Anspruch 1 als eine viskose Flüssigkeit ohne Verwendung der Mitsunobu-Reaktion herzustellen.

10. Verfahren nach Anspruch 9, wobei n-Propylalkohol mit Dichlormethan, Toluol, Benzol und einer beliebigen Kombinationen davon gemischt wird.

11. Verfahren nach Anspruch 9, wobei die anorganische Säure Schwefelsäure ist.

12. Verfahren nach Anspruch 9, wobei das anorganische Salz Phosphoroxychlorid ist.

13. Verfahren nach Anspruch 9, wobei der Ertrag an 2,2-Diphenyl-2-propyloxypropylacetat-7 mindestens 90 % beträgt.

## Revendications

1. Procédé de synthèse de chlorhydrate de propivérine, qui comprend
la réaction de l'acétate de 2,2-diphényl-2-propyloxy propyle 7 avec le N-méthyl-4-pipéridinol et un catalyseur comprenant le t-butylate de potassium ou de sodium, avec un catalyseur de transfert de phase sous agitation constante sous un gaz inerte pendant une longue durée, à la fin de ladite réaction, un chlorure inorganique est ajouté pour neutraliser le pH, ladite réaction étant soumise à un vide pour éliminer le N-méthyl-4-pipéridinol en excès afin d'obtenir un résidu, ledit résidu étant ensuite extrait dans un premier solvant organique et lavé avec de l'eau, ladite extraction étant suivie d'un traitement du résidu sous pression réduite pour éliminer le solvant organique afin d'obtenir un produit brut, qui est la base de propivérine, ledit produit brut étant ensuite traité par dissolution dans un second solvant organique avec du charbon actif et avec de l'acide chlorhydrique pour donner le chlorhydrate de propivérine.

2. Procédé selon la revendication 1, dans lequel le catalyseur de transfert de phase est choisi dans le groupe constitué du bromure de tétrabutylammonium, du bromure de tétraphosphonium, du polyéthylène glycol, des sels d'ammonium et des sels de phosphonium.

3. Procédé selon la revendication 1 ou la revendication 2, dans lequel la réaction de l'acétate de 2,2-diphényl-2-propyloxy propyle 7 avec le N-méthyl-4-pipéridinol est réalisée à température ambiante.

4. Procédé selon la revendication 1, dans lequel le gaz inerte est choisi parmi l'azote, l'argon, l'hélium.

5. Procédé selon la revendication 1, dans lequel l'agitation est réalisée pendant une durée de 1 à 3 jours.

6. Procédé selon la revendication 1, dans lequel le chlorure inorganique est le chlorure d'ammonium pour neutraliser le milieu réactionnel.

7. Procédé selon la revendication 1, dans lequel le premier solvant organique est le toluène.

8. Procédé selon la revendication 1, dans lequel le second solvant organique est l'acétone.

9. Procédé selon la revendication 1, qui comprend la réaction de l'acide benzilique 1 et de l'alcool n-propylique en présence d'une ou de plusieurs substances choisies parmi un réactif de type acide inorganique, un réactif organique, un sel inorganique et le chlorure de thionyle, ledit procédé comprenant en outre le reflux de l'acide benzilique 1 dans l'alcool n-propylique en présence de la substance ou des substances choisies parmi un réactif de type acide inorganique, un réactif organique, un sel inorganique ou le chlorure de thionyle à un rapport molaire de 10 % à 200 %, pour produire l'acétate de 2,2-diphényl-2-propyloxy propyle 7 de la revendication 1 sous la forme d'un liquide visqueux, sans utiliser la réaction de Mitsunobu.

10. Procédé selon la revendication 9, dans lequel l'alcool n-propylique est mélangé avec du dichlorométhane, du toluène, du benzène et l'une quelconque de leurs combinaisons.

11. Procédé selon la revendication 9, dans lequel l'acide inorganique est l'acide sulfurique.

12. Procédé selon la revendication 9, dans lequel l'acide inorganique est l'oxychlorure de phosphore.

13. Procédé selon la revendication 9, dans lequel le rendement en acétate de 2,2-diphényl-2-propyloxy propyle 7 est au moins de 90 %.
